# EUROPÄISCHE PATENTSCHRIFT

(11) **EP 3 391 924 B1**
(45) Veröffentlichungstag und Bekanntmachung des Hinweises auf die Patenterteilung: **30.12.2020**
(21) Anmeldenummer: 18000374.1
(22) Anmeldetag: 18.04.2018
(51) Int. Cl.: A61M 16/00

(54) **BEATMUNGSVORRICHTUNG**
VENTILATION APPARATUS
DISPOSITIF DE RESPIRATION

(30) Priorität: 20.04.2017 DE 102017003857
(43) Veröffentlichungstag der Anmeldung: 24.10.2018
(73) Patentinhaber: Löwenstein Medical Technology S.A., 2557 Luxembourg (LU)
(72) Erfinder: Göbel, Christof, 22547 Hamburg (DE); Heinrich, Wolfram, 22523 Hamburg (DE)
(74) Vertreter: Marx, Thomas

(56) Entgegenhaltungen:
- WO-A1-2011/050059
- WO-A1-2017/027906
- DE-A1- 4 018 347
- DE-A1-102009 025 327
- DE-A1-102012 013 656
- JP-A- 2000 036 327
- US-A1- 2006 216 582
- US-A1- 2012 055 340

## Beschreibung

Die vorliegende Erfindung betrifft eine Beatmungsvorrichtung, umfassend wenigstens ein Beatmungsgerät mit wenigstens einer Beatmungseinrichtung zum Erzeugen wenigstens eines Atemluftstroms für eine Atemtherapie und wenigstens eine Energiespeichereinrichtung zum Bereitstellen der zum Betrieb benötigten Energie.

Beatmungsgeräte und insbesondere Schlaftherapiegeräte sind häufig mit einer Energiespeichereinrichtung, beispielsweise mit Akkus oder Batterien ausgestattet, um einen vom Stromnetz unabhängigen Beatmungsbetrieb bieten zu können. So ist ein Einsatz auch an Orten ohne erreichbare Steckdosen oder auf Reisen besonders einfach möglich.

Die WO2011/050059 A1 offenbart eine Atemmaske, mit einer integrierten Gebläseeinrichtung und einem Akku. Die Gebläseeinrichtung erzeugt einen Atemluftstrom und einen Kühlluftstrom, der den Akku kühlt.

Im Betrieb und auch während des Ladevorgangs tritt bei Akkus grundsätzlich eine Erwärmung auf. Allerdings darf der Akku gerade während der Ladung eine bestimmte Eigentemperatur in der Regel nicht überschreiten. Diese zulässige Eigentemperatur liegt häufig relativ niedrig und typischerweise bei ca. 45°C. Die sich einstellende Akkutemperatur ist u.a. abhängig vom Ladestrom bzw. von der Geschwindigkeit des Ladevorgangs. Zur Vermeidung höherer Temperaturen wird der Akku üblicherweise mit eher kleinen Ladeströmen und somit sehr langsam geladen. Daher dauert es dann entsprechend lange, bis eine vollständige Ladung erreicht ist. Das bringt erhebliche Einbußen in der täglichen Praxis für den Anwender mit sich.

Es ist daher die Aufgabe der vorliegenden Erfindung, den Ladevorgang einer Energiespeichereinrichtung eines Beatmungsgerätes zu verbessern und insbesondere zügiger und komfortabler für den Anwender zu gestalten.

Diese Aufgabe wird gelöst durch eine Beatmungsvorrichtung mit den Merkmalen des Anspruchs 1. Bevorzugte Weiterbildungen der Erfindung sind Gegenstand der Unteransprüche. Weitere Vorteile und Merkmale der vorliegenden Erfindung ergeben sich aus der allgemeinen Beschreibung und der Beschreibung der Ausführungsbeispiele.

Die erfindungsgemäße Beatmungsvorrichtung bietet viele Vorteile. Einen erheblichen Vorteil bietet die Kühleinrichtung, mit der die Energiespeichereinrichtung beim Ladevorgang gekühlt werden kann. Dadurch kann die Eigentemperatur der Energiespeichereinrichtung abgesenkt bzw. entsprechend niedrig gehalten werden. So ist ein Aufladen der Energiespeichereinrichtung mit höheren Ladeströmen möglich, ohne dass es zu einer ungünstigen Wärmeentwicklung kommt. Die erfindungsgemäße Beatmungsvorrichtung bietet somit erheblich verkürzte Ladezeiten und ermöglicht dem Anwender einen erheblichen Komfortgewinn für die tägliche Praxis seiner Atemtherapie bzw. mit seinem Schlaftherapie- oder Beatmungsgerät.

Die Energiespeichereinrichtung umfasst insbesondere wenigstens eine Zelleneinheit und wenigstens eine Wärmeleitende mit der Zelleneinheit gekoppelte Wandung. Insbesondere ist mittels des Kühlluftstroms Wärme von der Wandung abführbar. Insbesondere verläuft der Kühlluftstrom entlang wenigstens eines Teils der Wandung. Anhand einer solchen Wandung kann die Energiespeichereinrichtung besonders gut gekühlt werden. Insbesondere ist die Wand als ein Wärmetauscher konzipiert oder stellt wenigstens einen solchen bereit.

Die Wandung ist insbesondere Teil eines Gehäuses der Energiespeichereinrichtung und insbesondere eines Gehäuses einer Akkueinheit der Energiespeichereinrichtung. Die Wandung kann auch ein Gehäuse bereitstellen oder mit einem solchen wärmeleitend verbunden sein. Insbesondere besteht die Wandung aus einem gut wärmeleitenden Werkstoff, z. B. einer Metalllegierung bzw. einem Metallwerkstoff. Die Kopplung der Zelleneinheit mit der Wandung kann durch einen direkten Kontakt und/oder durch einen mittelbaren Kontakt erfolgen. Bei mittelbarem Kontakt kann zwischen Zelleneinheit und Wandung wenigstens eine wärmeleitende Zwischenlage und/oder wenigstens ein wärmeleitendes Medium angeordnet sein. Die Zelleneinheit kann auch berührend an der Wandung anliegen.

Die Energiespeichereinrichtung, insbesondere wenigstens eine Akkueinheit der Energiespeichereinrichtung, ist insbesondere mit einer im Verhältnis zum Volumen großen bzw. optimierten Oberfläche ausgestattet, sodass entlang der Wandung eine besonders gute Wärmeabfuhr möglich ist. Insbesondere umfasst die Wandung wenigstens eine Einrichtung zur Oberflächenvergrößerung. Die Wandung umfasst dazu beispielsweise wenigstens eine Kühlrippe und/oder wenigstens eine Lamelle und/oder wenigstens eine andere geeignete Struktur zur Oberflächenvergrößerung.

Die Energiespeichereinrichtung umfasst insbesondere eine Mehrzahl von Zelleneinheiten. Dabei ist vorzugsweise jede Zelleneinheit direkt und/oder mittelbar mit wenigstens einer Wandung wärmeleitend gekoppelt. Vorzugsweise umfasst die Energiespeichereinrichtung wenigstens eine Akkueinheit, welche eine Mehrzahl von Zelleneinheiten aufweist, welche von wenigstens einer Wandung umgeben sind.

Insbesondere umfasst die Energiespeichereinrichtung wenigstens eine im Beatmungsgerät aufnehmbare interne Akkueinheit. Insbesondere ist mittels des Kühlluftstroms Wärme von wenigstens einer Wandung der internen Akkueinheit abführbar. Möglich ist auch, dass mittels des Kühlluftstroms Wärme von wenigstens einem Geräteabschnitt des Beatmungsgerätes abführbar ist. Der Geräteabschnitt ist dabei vorzugsweise wärmeleitend mit wenigstens einer Wandung der internen Akkueinheit gekoppelt. Insbesondere ist die interne Akkueinheit von wenigstens einer Außenseite des Beatmungsgerätes zugänglich und vorzugsweise entnehmbar. Die interne Akkueinheit ist insbesondere in das Beatmungsgerät integriert bzw. in einem Inneren des Beatmungsgerätes aufgenommen. Es ist möglich, dass die interne Akkueinheit auswechselbar gestaltet ist.

Die Wandung ist in einer Ausführung zumindest eine innere Wandung bzw. eine Innenwand der Akkueinheit. Der Geräteabschnitt ist insbesondere in das Innere des Beatmungsgerätes gerichtet. Der Geräteabschnitt kann zum Beispiel eine Innenwand eines Gehäuses des Beatmungsgerätes sein. Erfindungsgemäß strömt Atemluft und/oder Kühlluft während des Betriebs an der Innenwandung vorbei, so dass an dieser Wandung ein Wärmeaustausch stattfindet.

Besonders bevorzugt umfasst die Beatmungsvorrichtung wenigstens einen Transportbehälter zur Aufnahme des Beatmungsgerätes. Der Transportbehälter ist insbesondere als wenigstens eine Tasche ausgebildet oder umfasst wenigstens eine solche. Insbesondere umfasst der Transportbehälter wenigstens einen Einlass und/oder wenigstens einen Auslass für den Kühlluftstrom. Insbesondere stellt der Transportbehälter zwischen Einlass und Auslass wenigstens eine Strömungsverbindung für den Kühlluftstrom bereit. Insbesondere verläuft die Strömungsverbindung wenigstens abschnittsweise entlang wenigstens eines Teils der Energiespeichereinrichtung und besonders bevorzugt entlang wenigstens einer Wandung der Energiespeichereinrichtung oder auch durch diese hindurch.

Eine solche Ausgestaltung ist besonders vorteilhaft, da das Beatmungsgerät sicher und komfortabel transportiert und zudem auch direkt im Transportbehälter aufgeladen werden kann. Besonders vorteilhaft ist zudem, dass auch schnellere Ladevorgänge bzw. Ladevorgänge mit höheren Ladeströmen aufgrund des Kühlluftstroms im Transportbehälter problemlos möglich sind. So kann der Anwender sein Beatmungsgerät einfach in der Tasche belassen und darin zügig aufladen, wenn er auf Reisen eine Steckdose zur Verfügung hat. Die Erfindung bietet hier erhebliche Vorteile gegenüber dem Stand der Technik, da aufgrund der üblicherweise hohen Wärmeübergangswiderstände von Transportbehältern bzw. Akkugehäuse nur ein sehr langsames Aufladen der Energiespeichereinrichtung erfolgen kann, solange sich das Beatmungsgerät in dem Transportbehälter befindet. Der Transportbehäter stellt in einer bevorzugten Ausführung eine Tasche dar.

Insbesondere ist das Beatmungsgerät und vorzugsweise die Beatmungseinrichtung in dem Transportbehälter betreibbar, sodass das Beatmungsgerät auch zur Beatmung in dem Transportbehälter verbleiben kann. Der Transportbehälter ist insbesondere auch ein Aufbewahrungsbehälter und Betriebsbehälter. Der Transportbehälter kann wenigstens ein Fach und/oder wenigstens eine Abtrennung für wenigstens eine Akkueinheit und/oder für das Beatmungsgerät aufweisen. Insbesondere ist der Kühlluftstrom durch den Einlass ansaugbar und/oder über den Auslass aus dem Transportbehälter ausblasbar. Der Kühlluftstrom wird ferner innerhalb des Transportbehälters vom Atemlufteinlass 51 der Beatmungsvorrichtung aufgenommen und verlässt diese über den Atemluftauslass 61. Die Strömungsverbindung kann wenigstens abschnittsweise wenigstens einen Strömungskanal aufweisen. Der Strömungskanal kann einen teilweise offenen und/oder einen teilweise geschlossenen Umfang aufweisen. Möglich ist auch, dass zwischen Einlass und Auslass wenigstens abschnittsweise eine im Wesentlichen frei im Transportbehälter verlaufende Strömungsverbindung vorgesehen ist.

Vorzugsweise umfasst die Energiespeichereinrichtung wenigstens eine an das Beatmungsgerät anschließbare externe Akkueinheit. Insbesondere ist die externe Akkueinheit im Transportbehälter aufnehmbar. Insbesondere verläuft die Strömungsverbindung wenigstens abschnittsweise entlang wenigstens eines Teils der externen Akkueinheit und besonders bevorzugt entlang wenigstens einer Wandung der externen Akkueinheit. So kann auch die externe Akkueinheit aktiv gekühlt werden. Die Wandung ist insbesondere wie zuvor beschrieben wärmeleitend mit wenigstens einer Zelleneinheit gekoppelt.

Die externe Akkueinheit ist insbesondere außerhalb des Beatmungsgerätes angeordnet. Insbesondere ist die externe Akkueinheit über wenigstens eine Kabelverbindung mit dem Beatmungsgerät verbunden. Die Beatmungsvorrichtung kann auch zwei oder drei oder mehr externe Akkueinheiten umfassen.

Der Transportbehälter kann wenigstens ein Fach für die externe Akkueinheit umfassen, welches an wenigstens einer Seite und vorzugsweise an wenigstens zwei Seiten oder an mehr Seiten von dem Kühlluftstrom umströmbar ist.

In einer vorteilhaften Ausgestaltung umfasst die Energiespeichereinrichtung wenigstens eine in dem Beatmungsgerät aufnehmbare interne Akkueinheit. Dabei ist bevorzugt, dass die Strömungsverbindung wenigstens abschnittsweise entlang wenigstens einer zu einer Außenseite des Beatmungsgerätes gerichteten Wandung der internen Akkueinheit verläuft. Möglich ist auch, dass die Strömungsverbindung wenigstens abschnittsweise entlang wenigstens eines nach außen gerichteten Geräteabschnitts des Beatmungsgerätes verläuft, wobei der Geräteabschnitt wärmeleitend mit der Wandung verläuft. Insbesondere ist der Geräteabschnitt wärmeleitend mit der Wandung der internen Akkueinheit gekoppelt. So wird auch die interne Akkueinheit durch die im Transportbehälter strömende Luft gekühlt.

Die Wandung ist dabei insbesondere eine äußere Wandung bzw. eine Außenwand der internen Akkueinheit. Die Wandung kann aber auch eine Innenwand sein. Der Geräteabschnitt ist insbesondere zu einer Außenseite des Beatmungsgerätes gerichtet. Der Geräteabschnitt kann zum Beispiel Teil eines Gehäuses des Beatmungsgerätes sein. Der Geräteabschnitt kann auch wärmeleitend mit einem Gehäuse des Beatmungsgerätes gekoppelt sein und beispielsweise berührend an diesem angeordnet sein.

Insbesondere ist die externe Akkueinheit in Strömungsrichtung des Kühlluftstroms vor der internen Akkueinheit angeordnet. Möglich ist auch eine umgekehrte Anordnung.

Es ist möglich, dass die interne Akkueinheit wenigstens eine in ein Inneres des Beatmungsgerätes gerichtete Wandung umfasst. Das hat den Vorteil, dass die interne Akkueinheit sowohl an einer in das Innere des Beatmungsgerätes weisenden Wandung als auch an einer zur Außenseite des Beatmungsgerätes gerichteten Wandung gekühlt wird. Vorzugsweise ist mittels wenigstens eines weiteren Kühlluftstroms Wärme von dieser Wandung abführbar. In einer bevorzugten Ausführung wird der Kühlluftstrom von der primären Gebläseeinrichtung erzeugt, die im Therapiemodus der Beatmungsvorrichtung die Atemluft zum Patienten transportiert. In einer alternativen oder ergänzenden Ausführung umfasst die Kühleinrichtung zur Erzeugung des Kühlluftstroms wenigstens eine weitere Gebläseeinrichtung.

Die weitere Gebläseeinrichtung (Sekundärlüfter) ist beispielsweise als ein Axiallüfter ausgebildet oder umfasst wenigstens einen solchen. Der Sekundärlüfter ist insbesondere auch zur Lüftung bzw. Kühlung wärmeempfindlicher Komponenten und beispielsweise von Elektronikkomponenten des Beatmungsgerätes vorgesehen. Beispielsweise kann die weitere Gebläseeinrichtung zugeschaltet werden, wenn der Kühlluftstrom der Gebläseeinrichtung nicht ausreicht oder wenn die Kühlung anderer elektronischer Komponenten erfoderlich ist. Der weitere Kühlluftstrom innerhalb des Therapiegerätes ist bevorzugt separat zum Atemluftstrom und/oder zum Kühlluftstrom geführt. Möglich ist auch eine wenigstens abschnittsweise gemeinsame Führung des weiteren Kühlluftstroms mit dem Atemluftstrom und/oder dem Kühlluftstrom. Der weitere Kühlluftstrom hat typischerweise einen vom primären Kühlluftstrom und/oder Atemluftstrom separaten Auslass. Der weitere Kühlluftstrom hat darüber hinaus in einer bevorzugten Ausführungsform einen separaten Einlass (nicht gezeigt).

Die Kühlung der in das Innere des Beatmungsgerätes gerichteten Wandung kann aber auch mittels des primären Kühlluftstroms und/oder der Gebläseeinrichtung erfolgen. In einer solchen Ausgestaltung wird ein Kühlluftstrom beispielsweise sowohl entlang der zur Außenseite als auch entlang der in das Innere gerichteten Wandung der internen Akkueinheit vorbeigeführt.

In allen Ausgestaltungen ist es besonders bevorzugt, dass die Gebläseeinrichtung dazu geeignet und ausgebildet ist, den Atemluftstrom für die Atemtherapie wenigstens teilweise zu erzeugen, sodass der Atemluftstrom wenigstens zeitweise als Kühlluftstrom einsetzbar ist. Das bietet den erheblichen Vorteil, dass zur Kühlung und Beatmung eine gemeinsame Gebläseeinrichtung eingesetzt wird. Insbesondere sind dadurch typischerweise größere Kühlluftströme zu erzielen - verglichen mit einer ausschließlichen Kühlung über einen im Bereich der Elektronik angeordneten Kühllüfter (Axiallüfter). Somit lässt sich erfindungsgemäß eine effektivere Wärmeabfuhr im Bereich der Energiespeichereinrichtung erzielen.

Erfindungsgemäß ist die Gebläseeinrichtung auch der Beatmungseinrichtung zugeordnet Der Atemluftstrom entspricht wenigstens abschnittsweise dem Kühlluftstrom. Erfindungsgemäß umfasst die Beatmungseinrichtung die Gebläseeinrichtung, welche zur Erzeugung des Kühlluftstroms vorgesehen ist. Insbesondere sind mittels der Gebläseeinrichtung Kühlluftstrom und Atemluftstrom zeitgleich und/oder zeitversetzt erzeugbar.

Vorzugsweise ist der Kühlluftstrom durch wenigstens einen Atemlufteinlass in das Beatmungsgerät ansaugbar. Erfindungsgemäß ist der Kühlluftstrom über wenigstens einen Atemluftauslass aus dem Beatmungsgerät abführbar. So ist eine besonders kostengünstige und konstruktiv unaufwendige Umsetzung möglich. Insbesondere ist die Beatmungseinrichtung dazu geeignet und ausgebildet, den zur Beatmung benötigten Atemluftstrom über den Atemlufteinlass anzusaugen und/oder über den Atemluftauslass abzuführen. Der Atemluftauslass ist insbesondere zur Kopplung mit wenigstens einem Beatmungsschlauch ausgebildet. Der Atemluftauslass kann wenigstens einen Beatmungsschlauch umfassen, sodass der Kühlluftstrom über den Beatmungsschlauch abführbar ist. Vorzugsweise erfolgt die Abfuhr des Kühlluftstroms durch den Beatmungsschlauch, ohne dass ein Patienteninterface bzw. eine Atemschnittstelle gekoppelt ist.

Besonders bevorzugt verläuft der Atemluftauslass wenigstens teilweise durch den Auslass des Transportbehälters oder wird wenigstens teilweise durch diesen bereitgestellt. Wiederum bevorzugt stellt der Auslass des Transportbehälters die Öffnung für den Atemluftauslass des Therapiegerätes dar. Der Atemluftauslass kann wenigstens teilweise mit dem Auslass des Transportbehälters strömungsverbunden sein. Besonders bevorzugt ist der Atemlufteinlass wenigstens teilweise mit dem Einlass des Transportbehälters strömungsverbunden. Besonders bevorzugt wird der Atemluftstrom wenigstens abschnittsweise durch die vom Transportbehälter bereitgestellte Strömungsverbindung geführt. Besonders bevorzugt sind der Atemluftstrom und der Kühlluftstrom wenigstens abschnittsweise durch eine gemeinsame Strömungsverbindung im Beatmungsgerät geführt. So wird die Anzahl von Luftöffnungen bzw. Strömungsverbindungen reduziert und eine kompakte und kostengünstige Ausgestaltung erreicht.

Erfindungsgemäß umfasst die Beatmungsvorrichtung wenigstens eine Steuereinrichtung. Die Steuereinrichtung ist insbesondere dazu geeignet und ausgebildet, bei Nichtbetrieb der Beatmungseinrichtung mittels der Ladeeinrichtung wenigstens einen Lademodus zum beschleunigten Aufladen der Energiespeichereinrichtung durchzuführen. Die Beatmungseinrichtung ist insbesondere dazu geeignet und ausgebildet, zur Durchführung des Lademodus zum beschleunigten Aufladen mittels der Gebläseeinrichtung wenigstens einen Kühlluftstrom mit wenigstens einer definierten Durchflussrate zu erzeugen, um eine ausreichende Kühlung während des beschleunigten Aufladens zu gewährleisten. Im Lademodus zum beschleunigten Aufladen erfolgt insbesondere eine Ladung der Energiespeichereinrichtung mit einer maximal vorgesehenen Ladeleistung.

Besonders bevorzugt erfolgt in dem Lademodus zum beschleunigten Aufladen die Erzeugung des Kühlluftstroms insbesondere mittels der Gebläseeinrichtung, welche auch zum Erzeugen des Atemluftstroms geeignet und ausgebildet ist. Die Durchflussrate ist beispielsweise durch einen Wert für einen Volumenstrom und/oder für einen Massenstrom definiert.

Da die Gebläseeinrichtung im Nichtbetrieb der Beatmungseinrichtung für die Beatmung nicht benötigt wird, kann diese besonders gut auf den Kühlluftstrom mit der definierten Durchflussrate eingestellt werden. So steht für das beschleunigte Aufladen eine besonders hochwertige und geräuscharme Gebläseeinrichtung zur Verfügung, ohne das weitere Lüfter oder dergleichen benötigt werden.

Ein Nichtbetrieb der Beatmungseinrichtung liegt insbesondere dann vor, wenn kein Atemluftstrom für die Atemtherapie erzeugt wird bzw. keine Atemtherapie stattfindet. Insbesondere liegt bei einem Standby des Beatmungsgerätes ein Nichtbetrieb der Beatmungseinrichtung vor.

Insbesondere ist die Steuereinrichtung dazu geeignet und ausgebildet, mittels der Ladeeinrichtung wenigstens einen Normallademodus zum Aufladen der Energiespeichereinrichtung durchzuführen. Der beschleunigte Lademodus ist insbesondere dazu geeignet und ausgebildet, die Energiespeichereinrichtung in einer kürzeren Zeit als der Normallademodus vollständig zu laden. Insbesondere benötigt der Lademodus zum beschleunigten Aufladen maximal 75 % der Zeit und vorzugsweise maximal die Hälfte der Zeit des Normallademodus für eine vollständige Ladung der Energiespeichereinrichtung. Möglich ist auch, dass der Lademodus zum beschleunigten Aufladen nur maximal ein Viertel oder weniger der Zeit des Normallademodus benötigt. Im Normallademodus kann ein reduzierter oder auch gar kein Kühlluftstrom vorgesehen sein. Möglich ist auch, dass im Normallademodus nur der weitere Kühlluftstrom der weiteren Gebläseeinrichtung vorgesehen ist.

Die Steuereinrichtung kann dazu geeignet und ausgebildet sein, den Lademodus zum beschleunigten Aufladen automatisch und beispielsweise bei Nichtbetrieb der Beatmungseinrichtung automatisch einzustellen. Möglich ist auch, dass die Einstellung des Lademodus zum beschleunigten Aufladen wenigstens eine Benutzereingabe erfordert. Möglich ist auch, dass die Steuereinrichtung dazu geeignet und ausgebildet ist, den Lademodus zum beschleunigten Aufladen in Abhängigkeit eines Ladezustands der Energiespeichereinrichtung und/oder in Abhängigkeit einer Verfügbarkeit eines Ladestroms einer Energieversorgung einzustellen.

Die Steuereinrichtung ist vorzugsweise dazu geeignet und ausgebildet, den Lademodus zum beschleunigten Aufladen der Energiespeichereinrichtung auch bei Betrieb der Beatmungseinrichtung auszuführen, wenn eine Durchflussrate des dabei erzeugten Atemluftstroms die definierte Durchflussrate erreicht und/oder überschreitet, sodass der Atemluftstrom als Kühlluftstrom einsetzbar ist und eine ausreichende Kühlung während des beschleunigten Aufladens gewährleistet. In dieser Ausgestaltung ist der Atemluftstrom insbesondere als Kühlluftstrom einsetzbar. Dadurch ist ein zügiges Aufladen auch während einer Beatmung möglich, sodass die Energiespeichereinrichtung besonders schnell wieder für einen mobilen Einsatz zur Verfügung steht.

Die Steuereinrichtung ist insbesondere dazu geeignet und ausgebildet, bei einer anderen Durchflussrate des Atemluftstroms als der definierten Durchflussrate den Normallademodus und/oder wenigstens einen Lademodus mit reduzierter Ladeleistung einzustellen.

Die definierte Durchflussrate entspricht vorzugsweise einem Luftvolumenstrom von wenigstens einem Liter pro Minute und besonders bevorzugt von wenigstens 5 l/min. Insbesondere entspricht die definierte Durchflussrate einem Luftvolumenstrom von maximal 100 l/min und vorzugsweise maximal 50 l/min und besonders bevorzugt maximal 30 l/min. Solche Durchflussraten bieten eine gute Kühlwirkung bei einem besonders geringen Energieverbrauch der Gebläseeinrichtung. Es ist möglich, dass die Steuereinrichtung dazu geeignet und ausgebildet ist, die Durchflussrate vorübergehend und beispielsweise für einige Minuten oder einige Sekunden auf einen Luftvolumenstrom von mehr als 100 l/min anzuheben. Dadurch kann eine vorübergehend höhere Wärmeentwicklung besonders gut kompensiert werden.

Der Lademodus zum beschleunigten Aufladen ist besonders bevorzugt durchführbar, wenn das Beatmungsgerät in dem Transportbehälter aufgenommen ist. Das ermöglicht eine komfortable Handhabung der Beatmungsgeräteeinrichtung und ein komfortables Aufladen der Energiespeichereinrichtung und bietet eine schnelle Wiedereinsatzbereitschaft der Beatmungseinrichtung nach einem verkürzten Ladevorgang. Durch die zuvor beschriebene Ausgestaltung des Transportbehälters und insbesondere durch dessen Strömungsverbindung ist eine solche Ausgestaltung besonders vorteilhaft umsetzbar.

In einer vorteilhaften Ausgestaltung ist die Steuereinrichtung dazu geeignet und ausgebildet, eine Durchflussrate des Kühlluftstroms in Abhängigkeit wenigstens einer vorgesehenen Ladeleistung der Ladeeinrichtung einzustellen. So kann bei einer höheren Ladeleistung durch einen größeren Kühlluftstrom eine ausreichende Kühlwirkung gewährleistet werden. Zugleich kann bei einer geringeren Ladeleistung der Energieverbrauch der Gebläseeinrichtung durch eine geringere Durchflussrate des Kühlluftstroms reduziert werden.

Die Steuereinrichtung kann auch dazu geeignet und ausgebildet sein, wenigstens eine Ladeleistung in Abhängigkeit wenigstens einer vorgesehenen Durchflussrate des Kühlluftstroms einzustellen. Die Steuereinrichtung ist dabei insbesondere die zuvor beschriebene Steuereinrichtung. So kann die Ladeleistung beispielsweise reduziert werden, wenn der Kühlluftstrom nur mit einer begrenzten Durchflussrate bereitgestellt werden kann. Das ist beispielsweise dann der Fall, wenn der Kühlluftstrom auch als Atemluftstrom eingesetzt wird oder wenn ein besonders geräuscharmer oder energiesparender Betrieb erfolgen soll.

Die Steuereinrichtung ist insbesondere dazu geeignet und so ausgebildet,dass eine Erfassung der Akkutemperatur durch Messung, im, am oder durch indirekte Messung in der Umgebung, möglich ist. Die Steuereinrichtung ist insbesondere dazu geeignet und ausgebildet, in Abhängigkeit der erfassten Temperatur die Ladeeinrichtung und/oder die Kühleinrichtung, insbesondere die Gebläseeinrichtung, wenigstens teilweise zu regeln. Durch eine solche Regelung ist in Abhängigkeit der erfassten Temperatur beispielsweise eine Durchflussrate des Kühlluftstroms einstellbar. Besonders vorteilhaft ist eine solche Regelung dann, wenn dabei eine geforderte Ladeleistung eingehalten werden soll, beispielsweise im Lademodus zum beschleunigten Aufladen. Durch eine solche Regelung kann aber auch in Abhängigkeit der erfassten Temperatur eine Ladeleistung einstellbar sein. Das ist dann besonders vorteilhaft, wenn eine geforderte Durchflussrate des Kühlluftstroms eingehalten werden soll, beispielsweise wenn eine Atemtherapie stattfindet und dabei ein genau definierter Atemluftstrom als Kühlluftstrom dient. Durch eine solche Regelung ergibt sicher weiterhin der Vorteil, dass für verschiedene Anordnungen der Beatmungsgeräteeinrichtung (z.B. Gerät in der Tasche oder Gerät freistehend) die jeweils schnellste Ladung durchgeführt werden kann, die die sich einstellende Temperatur der Energiespeichereinrichtung als führende bzw. limitierende Größe erlaubt.

Zur Erfassung der Temperatur ist die Steuereinrichtung insbesondere mit wenigstens einer Sensoreinrichtung wirkverbunden. Die Sensoreinrichtung ist insbesondere dazu geeignet und ausgebildet, wenigstens eine charakteristische Größe für eine Temperatur der Energiespeichereinrichtung zu erfassen.

Insbesondere ist in der Steuereinrichtung wenigstens ein Algorithmus zur Regelung der Ladeleistung und/oder der Durchflussrate des Kühlluftstroms in Abhängigkeit wenigstens eines Temperatursignals der Sensoreinrichtung hinterlegt. Insbesondere ist jede Akkueinheit der Energiespeichereinrichtung mit wenigstens einem Sensormittel zur Erfassung der Temperatur ausgestattet. Insbesondere ist die Steuereinrichtung dazu geeignet und ausgebildet, wenigstens ein Temperatursignal von jeweils wenigstens einer Akkueinheit zu erfassen. Es ist möglich, dass die Steuereinrichtung dazu geeignet und ausgebildet ist, in Abhängigkeit der erfassten Temperatur auch die weitere Gebläseeinrichtung wenigstens teilweise zu regeln.

Insbesondere ist die Kühleinrichtung dazu geeignet und ausgebildet, kühle Luft aus der Umgebung anzusaugen und an der Energiespeichereinrichtung vorbeizuleiten und vorzugsweise wieder an die Umgebung abzuführen bzw. aus dem Beatmungsgerät auszublasen.

Die Ladeeinrichtung umfasst insbesondere wenigstens eine Leistungselektronik zur Bereitstellung eines geeigneten Ladestroms bzw. einer geeigneten Ladespannung. Die Ladeeinrichtung kann auch wenigstens eine Überwachungseinrichtung zur Überwachung eines Ladezustands umfassen. Insbesondere ist die Ladeeinrichtung dazu geeignet und ausgebildet, einen Ladevorgang in Abhängigkeit eines Ladezustands der Energiespeichereinrichtung zu beginnen und/oder zu beenden. Vorzugsweise ist die Ladeeinrichtung mit wenigstens einem Energieversorgungsnetz verbindbar.

Die Steuereinrichtung ist alternativ oder ergänzend auch dazu geeignet und ausgebildet, Leistungs-Bedarfe anderer Leistungsverbraucher, wie beispielsweise Bedieneinrichtung, Display oder Anfeuchter oder Schlauchheizung, sowie die Temperatur der Energiespeichereinrichtung zu ermitteln und/oder zu berücksichtigen, und unter Berücksichtigung der zulässigen Temperatur der Energiespeichereinrichtung zumindest zeitweise eine für die Ladung der Energiespeichereinrichtung maximal verfügbare Leistung bereit zu stellen.

Weitere Vorteile und Merkmale der vorliegenden Erfindung ergeben sich aus der Beschreibung der Ausführungsbeispiele, welche mit Bezug auf die beiliegenden Figuren im Folgenden erläutert werden.

In der Figur zeigt:

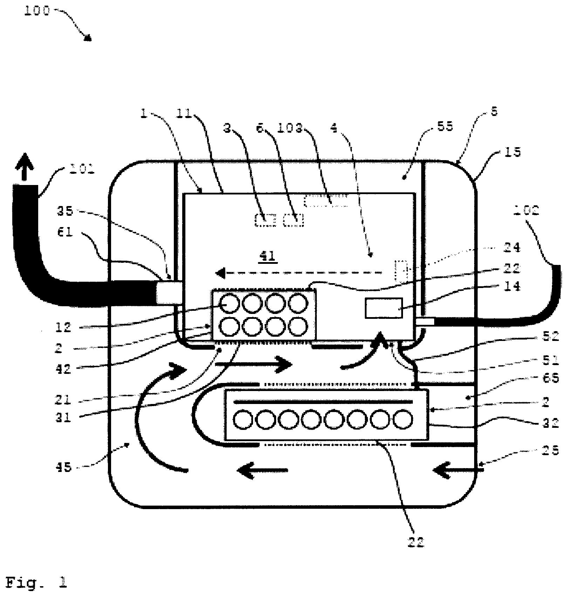

- Fig. 1: eine stark schematische Darstellung einer erfindungsgemäßen Beatmungsvorrichtung.

In der Figur 1 ist eine erfindungsgemäße Beatmungsvorrichtung 100 gezeigt, welche hier ein als Heimbeatmungsgerät oder Schlaftherapiegerät oder Notfallbeatmungsgerät eingesetztes Beatmungsgerät 1 umfasst. Das Beatmungsgerät 1 kann aber auch als klinisches Beatmungsgerät 1 ausgebildet sein.

Das Beatmungsgerät 1 umfasst eine Beatmungseinrichtung 11 zur Beatmung eines Anwenders bzw. Patienten mit einem Atemluftstrom. Der Atemluftstrom wird beispielsweise durch eine Gebläseeinrichtung 14 erzeugt. Die Gebläseeinrichtung 14 saugt dazu Luft aus der Umgebung über einen Atemlufteinlass 51 im Beatmungsgerät 1 an.

Für die Beatmung steuert die Beatmungseinrichtung 11 die Gebläseeinrichtung 14 so an, dass der Atemluftstrom einen bestimmten Beatmungsdruck und/oder einen bestimmten Luftvolumenstrom bzw. Flow aufweist.

Das Beatmungsgerät 1 ist mit einer hier nicht gezeigten Atemschnittstelle koppelbar, um den Atemluftstrom einem Patienten zur Beatmung zuzuführen. Die Atemschnittstelle kann beispielsweise als eine Nasalmaske, Vollgesichtsmaske, als ein Nasal-Pillow, als ein Tubus oder als eine Larynxmaske ausgestaltet sein.

Zur Verbindung der Atemschnittstelle mit der Beatmungseinrichtung 11 ist ein Beatmungsschlauch 101 vorgesehen. Der Beatmungsschlauch kann als heizbarer Schlauch ausgebildet sein. Der Beatmungsschlauch 101 wird über einen Atemluftauslass 61 mit der Beatmungseinrichtung 11 verbunden. Zwischen dem Atemluftauslass 61 und der Atemschnittstelle kann ein Ausatmungselement angeordnet sein, um ein Rückatmen von Kohlendioxid bzw. verbrauchter Luft in das Beatmungsgerät 1 zu verhindern.

Das Beatmungsgerät 1 kann z. B. als ein Fix-Level-Gerät oder auch als ein Automatik-Level-Gerät oder als Notfall- oder Heim- oder Klinik-Beatmungsgerät ausgebildet sein. Insbesondere erfolgt dabei durch die Beatmungseinrichtung 11 eine Regelung auf Soll-Geräteparameter, welche zuvor anhand der charakteristischen Atmung eines Benutzers individuell berechnet und festgelegt worden sind.

Das Beatmungsgerät 1 kann ein Display aufweisen. Im Bereich des Schlauchanschlusses kann ein Anfeuchter, welcher eine Heizung aufweisen kann, adaptiert werden.

Beispielsweise überwacht die Beatmungseinrichtung 11 die Druckverhältnisse an der Atemschnittstelle bzw. im Beatmungsschlauch 101 und regelt die Leistung der Gebläseeinrichtung 14 entsprechend nach, bis ein gewünschter Beatmungsdruck anliegt. So kann die Beatmungseinrichtung 11 mittels der Gebläseeinrichtung 14 Druckschwankungen kompensieren, die durch die Atemtätigkeit des Benutzers bedingt sind.

Die Beatmungseinrichtung 11 kann hier auch einen Atemphasenwechsel erkennen, sodass je nach Atemphase ein höherer bzw. niedrigerer Druck bereitgestellt werden kann. Beispielsweise kann das Beatmungsgerät 1 als ein CPAP- oder APAP-Gerät ausgebildet sein. Das Beatmungsgerät 1 kann auch als ein Bilevel-Gerät ausgebildet sein. Beispielsweise reagiert das Beatmungsgerät 1 auf bestimmte Atemereignisse, wie z. B. Schnarchen, Atemabflachungen und/oder obstruktive Druckspitzen mit entsprechenden Einstellungen der Beatmungs- bzw. Geräteparameter.

Die zur Einstellung der Beatmungseinrichtung 11 bzw. der Gebläseeinrichtung 14 benötigten Geräteparameter sowie die Gerätekonfiguration und/oder Gerätesoftware sind in der Beatmungseinrichtung 11 hinterlegt bzw. gespeichert.

Die Beatmungsvorrichtung 100 umfasst hier zudem eine Energiespeichereinrichtung 2 mit zwei Akkueinheiten 32, 42, um die für den Betrieb des Beatmungsgerätes 1 benötigte Energie unabhängig von einem Stromnetz bereitstellen zu können. Die Akkueinheiten 32,42 umfassen hier jeweils eine Mehrzahl von Zelleneinheiten 12, welche zur Erzeugung einer gewünschten Spannung entsprechend miteinander verschaltet sind.

Für einen Netzbetrieb bzw. zum Aufladen der Energiespeichereinrichtung 2 ist das Beatmungsgerät 1 zudem mit einem Netzkabel 102 ausgestattet. Das Aufladen der Energiespeichereinrichtung 2 erfolgt mittels einer Ladeeinrichtung 3, welche hier in dem Beatmungsgerät 1 integriert ist.

Zum Kühlen der Energiespeichereinrichtung 2 während Ladevorgängen und vorzugsweise auch während Entladevorgängen ist die Beatmungsvorrichtung 100 mit einer Kühleinrichtung 4 ausgestattet. Die Kühleinrichtung 4 erzeugt mittels der Gebläseeinrichtung 14 einen Kühlluftstrom, welcher entlang der Akkueinheiten 32, 42 strömt und dabei Wärme abgeführt. Der Kühlluftstrom ist hier durch Pfeile stark schematisch angedeutet.

Eine der beiden Akkueinheiten 32, 42 ist hier als eine interne Akkueinheit 42 innerhalb des Beatmungsgerätes 1 untergebracht bzw. in dieses integriert. Die andere Akkueinheit 32 ist hier als eine externe Akkueinheit 32 ausgebildet und über ein Anschlusskabel mit dem Beatmungsgerät 1 verbunden.

Die Akkueinheiten 32, 42 sind hier von jeweils wenigstens einer Wandung 22 umgeben, an welche die jeweiligen Zelleneinheiten 12 wärmeleitend angebunden sind. So wird die in den Zelleneinheiten 12 auftretende Wärme besonders gut abgeführt, wenn der Kühlluftstrom entlang der Wandungen 22 strömt. Die Wandung 22 kann beispielsweise Teil eines Gehäuses der Akkueinheiten 32, 42 sein. Die Zelleneinheiten 12 sind hier derart angeordnet, dass insbesondere jede Zelleneinheit 12 direkten Kontakt zur Wandung 22 besitzt. Die Akkueinheiten 32, 42 besitzen hier im Verhältnis zum Volumen bzw. zur Anzahl der verwendeten Zelleneinheiten 12 eine entsprechend große Oberfläche.

Die Beatmungsvorrichtung 100 umfasst hier zudem einen als Tasche 15 ausgebildeten Transportbehälter 5. In dem Transportbehälter 5 kann das Beatmungsgerät 1 sowohl zur Beatmung als auch zum Transport sowie auch während Ladevorgängen aufbewahrt werden.

Die Tasche 15 ist hier mit einem ersten Fach 55 für das Beatmungsgerät 1 und einem zweiten Fach 65 für die externe Akkueinheit 32 ausgestattet. Zudem weist die Tasche 15 hier einen Ausgang für das Netzkabel 102 und eine Durchführung für den Beatmungsschlauch 101 auf.

Der Kühlluftstrom wird hier erfindungsgemäß durch die Gebläseeinrichtung 14 bereitgestellt, welche auch den Atemluftstrom für die Beatmung erzeugt. Die Gebläseeinrichtung 14 saugt die Luft für den Kühlluftstrom über den Atemlufteinlass 51 in das Beatmungsgerät 1 ein und bläst den erwärmten Kühlluftstrom über den Atemluftauslass 61 bzw. einen daran gekoppelten Beatmungsschlauch 101 wieder aus. Dabei kann vorgesehen sein, dass der ausgeblasene Kühlluftstrom auch zur Beatmung und somit als Atemluftstrom eingesetzt wird.

Wenn das Beatmungsgerät 1 in der Tasche 15 aufgenommen ist, wird der Kühlluftstrom über einen in der Tasche 15 vorgesehenen Einlass 25 angesaugt. Zudem weist die Tasche 15 einen Auslass 35 auf, durch welchen hier der Atemluftauslass 61 bzw. der daran angekoppelte Beatmungsschlauch 101 verlaufen.

Zwischen dem Einlass 25 und dem Auslass 35 stellt die Tasche 15 hier eine Strömungsverbindung 45 für den Kühlluftstrom bereit. Wenn der Kühlluftstrom als Atemluftstrom eingesetzt wird, wird vorzugsweise auch der Atemluftstrom entlang dieser Strömungsverbindung 45 angesaugt.

Die Strömungsverbindung 45 wird hier teilweise um das Fach 65 für die externe Akkueinheit 32 herumgeführt. Dabei verläuft die Strömungsverbindung 45 entlang zweier gegenüberliegender Wandungen 22 der externen Akkueinheit 32. Damit der Kühlluftstrom die Wandungen 22 besser erreicht, kann das Fach 65 im Bereich der Wandungen 22 bevorzugt mit besonders wärmeleitenden Material ausgestattet sein. Eine solche Ausgestaltung ist hier durch eine gepunktete Linie angedeutet.Zudem verläuft die Strömungsverbindung 45 hier entlang einer Außenseite 21 eines Geräteabschnitts 31 des Beatmungsgerätes 1. Dieser Geräteabschnitt 31 ist mit den Zelleneinheiten 12 und/oder der Wandung 22 der internen Akkueinheit 42 wärmeleitend gekoppelt. Der Geräteabschnitt 31 kann auch durch die Wandung 22 der internen Akkueinheit 42 bereitgestellt werden. So erfolgt eine Kühlung der internen Akkueinheit 42 von einer Außenseite 21 aus, bevor der Kühlluftstrom in das Beatmungsgerät 1 eintritt. Um die Wärmeabfuhr zu unterstützen, kann das Fach 55 im Bereich des Geräteabschnitts 31 bzw. der Wandung 22 mit einem luftdurchlässigen und/oder wärmeleitenden Material ausgestattet sein.

In einer vorteilhaften Ausgestaltung ist auch eine Kühlung einer Wandung 22 der internen Akkueinheit 42 vorgesehen, welche in einem Inneren 41 des Beatmungsgerätes 1 angeordnet ist. So verfügt die interne Akkueinheit 42 also über eine ins Innere 41 gewandte Kühlfläche 22 und eine zur Außenseite 21 des Beatmungsgerätes 1 gewandte Kühlfläche 22.

Im Inneren 41 des Beatmungsgerätes kann eine weitere wärmetauschende Fläche für den Kühlluftstrom bereit gestellt werden, welche hier durch einen gestrichelten Pfeil angedeutet ist. Der weitere Kühlluftstrom wird dabei bevorzugt von einer weiteren Gebläseeinrichtung 24 erzeugt und entlang der ins Innere 41 gerichteten Wandung 22 geblasen oder auch gesaugt. Die weitere Gebläseeinrichtung 24 ist zum Beispiel Teil einer geräteinternen Kühlung, beispielsweise für Elektronikkomponenten oder andere wärmeempfindliche Bauteile. So kann gleichzeitig oder alternativ zu der Gebläseeinrichtung 14 die weitere Gebläseeinrichtung 24 hinzugeschaltet werden, sodass bedarfsweise eine zusätzliche Kühlung der internen Akkueinheit 42 möglich ist. In einer alternativen Ausführungsform kann auch der Atemluftstrom oder Kühlluftstrom, der von der primären Gebläseeinheit (14) bereitgestellt wird, an der nach innen weisenden wärmetauschenden Fläche der Akkueinheit (42) zum weiteren Wärmetransport vorbei geführt werden.

Die Bedienung und Einstellung des Beatmungsgerätes 1 erfolgt über eine Bedieneinrichtung 103 mit Bedienelementen und einer Anzeigeeinrichtung. Darüber können hier auch Informationen über den Ladezustand der Akkueinheiten 32, 42 angezeigt werden.

Die Beatmungsvorrichtung 100 umfasst zudem eine hier im Beatmungsgerät 1 integrierte Steuereinrichtung 6. Die Steuereinrichtung 6 ist mit der Ladeeinrichtung 3 und der Kühleinrichtung 4 sowie der Beatmungseinrichtung 11 wirkverbunden.

In einem Nichtbetrieb bzw. wenn keine Beatmung stattfindet, kann die Steuereinrichtung 6 eine Durchflussrate und zum Beispiel den Volumenstrom des Kühlluftstroms an eine geforderte Ladeleistung anpassen. Dazu steuert die Steuereinrichtung 6 die Gebläseeinrichtung 14 und optional die weitere Gebläseeinrichtung 24 entsprechend an.

Während die Beatmungseinrichtung 11 in Betrieb ist bzw. eine Beatmung stattfindet, wird die Gebläseeinrichtung 14 vorrangig auf den für die Beatmung geforderten Druck- und/oder Flow bzw. Volumenstrom eingestellt. Die Steuereinrichtung 6 stellt dann die Ladeleistung so ein, dass die Akkueinheiten 32, 42 ausreichend mit dem zur Beatmung vorgesehenen Atemluftstrom gekühlt werden können. Der Atemluftstrom wird dann also als Kühlluftstrom eingesetzt.

Die Steuereinrichtung 6 ist hier zudem dazu geeignet und ausgebildet, einen Lademodus zum beschleunigten Aufladen bzw. einen Schnelllademodus durchzuführen. Der Schnelllademodus ist hier im Nichtbetrieb der Beatmungseinrichtung 11 bzw. im Standby, also wenn keine Therapie läuft und keine Atemluftströmung erzeugt wird, aktivierbar. Im Schnelllademodus wird die Ladeeinrichtung 3 mit erhöhter oder maximaler Ladeleistung betrieben.

Die Aktivierung kann beispielsweise automatisch passieren, wenn ein niedriger Ladezustand der Energiespeichereinrichtung 2 erreicht wird. Der Schnelllademodus kann auch durch eine Eingabe an der Bedieneinrichtung 103 erfolgen. Über die Bedieneinrichtung 103 kann auch wenigstens ein anderer Lademodus eingestellt werden.

Die Steuereinrichtung 6 stellt im Standby bzw. im Nichtbetrieb der Beatmungseinrichtung 11 einen sogenannten Low-Flow-Modus ein. Dazu wird der Kühlluftstrom auf eine definierte Durchflussrate eingestellt. Die definierte Durchflussrate weist z. B. einen Luftvolumenstrom zwischen 5 l/min und 50 l/min oder auch zwischen 5 l/min und 30 l/min auf. Bei solchen pneumatischen Lasten verbraucht die Gebläseeinrichtung 14 nur entsprechend wenig elektrische Leistung (typischerweise nur wenige Watt), insbesondere, wenn in dieser Betriebsweise der Luftvolumenstrom am Auslass 35 oder am Ende des Beatmungsschlauches 101 frei abströmen kann. Zudem ist eine solche Durchflussrate besonders gut geeignet, um einen ausreichend kräftigen Kühlluftstrom für eine effektive und zuverlässige Kühlung auch im Schnelllademodus zu erreichen.

Im Schnelllademodus wird der Kühlluftstrom mit der definierten Durchflussrate von der Gebläseeinrichtung 14 in die Tasche 15 gesogen. Der Kühlluftstrom wird dabei wie zuvor beschrieben entlang der Strömungsverbindung 45 durch die Tasche 15 geführt und zur Kühlung der Akkueinheiten 32, 42 eingesetzt. Die angesaugte Luft bzw. der erwärmte Kühlluftstrom wird über den Auslass 35 bzw. den Atemluftauslass 61 wieder in die Umgebung abgeführt.

Die Steuereinrichtung 6 kann hier die Durchflussrate des Kühlluftstroms auch auf einer Akkutemperatur basierend regeln. In einer solchen Betriebsweise wird der Akku 32, 42 gerade so schnell geladen, wie es die Betriebssituation thermisch erlaubt. Dabei kann vorgesehen sein, dass in der Steuereinrichtung 6 ein Kühlalgorithmus hinterlegt ist, der seine Temperatursignale von der jeweils zu ladenden Akkueinheit 32, 42 erhält.

Wenn während des Betriebs der Beatmungseinrichtung 11 bzw. während der Beatmung ein Atemluftstrom mit einer ausreichend hohen Durchflussrate erzeugt wird, kann auch dann ein Schnelllademodus aktiviert werden.

Die hier vorgestellte Erfindung ermöglicht ein besonders zügiges Aufladen, bei dem die Akkueinheiten 32, 42 während eines Geräte-Standby bzw. außerhalb einer Beatmung mit höherer Ladeleistung als sonst vorgesehen aufgeladen werden. Dabei werden die zur Erzeugung des Atemluftstroms vorgesehenen Komponenten auch zur Erzeugung eines Kühlluftstroms zur Kühlung der Akkueinheiten 32, 42 eingesetzt. Zudem erlaubt die hier vorgestellte Erfindung ein zügiges Aufladen auch dann, wenn das Beatmungsgerät 1 in einer Tasche 15 ist. Es kann sogar eine externe Akkueinheit 32 in der Tasche zügig aufgeladen und dabei zuverlässig und energiesparend sowie geräuscharm gekühlt werden.

### Bezugszeichenliste:

- 1: **Beatmungsgerät**
- 2: Energiespeichereinrichtung
- 3: Ladeeinrichtung
- 4: Kühleinrichtung
- 5: Transportbehälter
- 6: Steuereinrichtung
- 11: Beatmungseinrichtung
- 12: Zelleneinheit
- 14: Gebläseeinrichtung
- 15: Tasche
- 21: Außenseite
- 22: Wandung
- 24: Gebläseeinrichtung
- 25: Einlass
- 31: Geräteabschnitt
- 32: Akkueinheit
- 35: Auslass
- 41: Inneres
- 42: Akkueinheit
- 45: Strömungsverbindung
- 51: Atemlufteinlass
- 52: Anschlusskabel
- 55: Fach
- 61: Atemluftauslass
- 65: Fach
- 100: Beatmungsvorrichtung
- 101: Beatmungsschlauch
- 102: Netzkabel
- 103: Bedieneinrichtung

## Patentansprüche

1. Beatmungsvorrichtung (100), umfassend wenigstens ein Beatmungsgerät (1) mit wenigstens einer Beatmungseinrichtung (11), mit einer Gebläseeinrichtung (14) zum Erzeugen wenigstens eines Atemluftstroms für eine Atemtherapie und mit wenigstens einer aufladbaren Energiespeichereinrichtung (2) zum Bereitstellen der zum Betrieb benötigten Energie und mit wenigstens einer Ladeeinrichtung (3) zum Aufladen der Energiespeichereinrichtung (2), und mit einer Steuereinrichtung (6) zur Regelung der Gebläseeinrichtung (14), wobei zur Kühlung der Energiespeichereinrichtung (2) während eines Ladevorgangs wenigstens eine Kühleinrichtung (4) mit der Gebläseeinrichtung (14) zur Erzeugung wenigstens eines Kühlluftstroms umfasst ist, sodass mittels des Kühlluftstroms Wärme von der Energiespeichereinrichtung (2) abführbar ist, wobei der Kühlluftstrom durch die Gebläseeinrichtung (14) bereitgestellt wird, welche auch den Atemluftstrom für die Beatmung erzeugt, **dadurch gekennzeichnet, dass** die Steuereinrichtung (6) ausgebildet ist Luftvolumenstrom die Gebläseeinrichtung (14) so zu regeln, dass der während des Ladevorgangs andauert und am Atemluftauslass (61) des Beatmungsgerätes (1) oder am patientenseitigen Ende des Atemschlauches (101) abströmt.

2. Beatmungsvorrichtung (100) nach dem Vorhergehenden Anspruch, wobei die Energiespeichereinrichtung (2) wenigstens eine Zelleneinheit (12) und wenigstens eine Wärme leitend mit der Zelleneinheit (12) gekoppelte Wandung (22) umfasst und wobei mittels des Kühlluftstroms Wärme von der Wandung (22) abführbar ist.

3. Beatmungsvorrichtung (100) nach einem der vorhergehenden Ansprüche, umfassend wenigstens einen Transportbehälter (5), insbesondere eine Tasche (15), zur Aufnahme des Beatmungsgerätes (1), wobei der Transportbehälter (5) wenigstens einen Einlass (25) und wenigstens einen Auslass (35) für den Kühlluftstrom und zwischen Einlass (25) und Auslass (35) wenigstens eine Strömungsverbindung (45) für den Kühlluftstrom bereitstellt, wobei die Strömungsverbindung (45) wenigstens abschnittsweise entlang wenigstens eines Teils der Energiespeichereinrichtung (2) verläuft.

4. Beatmungsvorrichtung (100) nach dem vorhergehenden Anspruch, wobei die Energiespeichereinrichtung (2) wenigstens eine an das Beatmungsgerät (1) anschließbare externe Akkueinheit (32) umfasst und wobei die externe Akkueinheit (32) im Transportbehälter (5) aufnehmbar ist und wobei die Strömungsverbindung (45) wenigstens abschnittsweise entlang wenigstens eines Teils der externen Akkueinheit (32) verläuft.

5. Beatmungsvorrichtung (100) nach einem der vorhergehenden Ansprüche, wobei das Beatmungsgerät (1) und die externe Akkueinheit (32) während des Ladevorgangs im Transportbehälter verbleiben.

6. Beatmungsvorrichtung (100) nach einem der beiden vorhergehenden Ansprüche, wobei die Energiespeichereinrichtung (2) wenigstens eine im Beatmungsgerät (1) aufnehmbare interne Akkueinheit (42) umfasst und wobei die Strömungsverbindung (45) wenigstens abschnittsweise entlang wenigstens einer zu einer Außenseite (21) des Beatmungsgeräts (1) gerichteten Wandung (22) der internen Akkueinheit (32) verläuft und/oder entlang wenigstens eines nach außen gerichteten Geräteabschnitts (31) des Beatmungsgerätes (1) verläuft, wobei der Geräteabschnitt (31) Wärme leitend mit der Wandung (22) der internen Akkueinheit (42) gekoppelt ist.

7. Beatmungsvorrichtung (100) nach einem der vorhergehenden Ansprüche, wobei die interne Akkueinheit (42) wenigstens eine in ein Inneres (41) des Beatmungsgeräts (1) gerichtete Wandung (22) umfasst und wobei mittels eines Kühlluftstroms Wärme von dieser Wandung (22) abführbar ist.

8. Beatmungsvorrichtung (100) nach dem vorhergehenden Anspruch, wobei die interne Akkueinheit (42) wenigstens eine in ein Inneres (41) des Beatmungsgeräts (1) gerichtete Wandung (22) umfasst und wobei mittels wenigstens eines weiteren Kühlluftstroms Wärme von dieser Wandung (22) abführbar ist und wobei die Kühleinrichtung (4) zur Erzeugung des weiteren Kühlluftstroms wenigstens eine weitere Gebläseeinrichtung (24) umfasst.

9. Beatmungsvorrichtung (100) nach einem der vorhergehenden Ansprüche, umfassend wenigstens eine Steuereinrichtung (6), welche dazu geeignet und ausgebildet ist, bei Nichtbetrieb der Beatmungseinrichtung (11) mittels der Ladeeinrichtung (3) wenigstens einen Lademodus zum beschleunigten Aufladen der Energiespeichereinrichtung (2) durchzuführen und dazu mittels der Gebläseeinrichtung (14) wenigstens einen Kühlluftstrom mit wenigstens einer definierten Durchflussrate zu erzeugen, um eine auseichende Kühlung während des beschleunigten Aufladens zu gewährleisten.

10. Beatmungsvorrichtung (100) nach dem vorhergehenden Anspruch, wobei die Steuereinrichtung (6) dazu geeignet und ausgebildet ist, den Lademodus zum beschleunigten Aufladen der Energiespeichereinrichtung (2) auch bei Betrieb der Beatmungseinrichtung (11) dann auszuführen, wenn eine Durchflussrate des dabei erzeugten Atemluftstroms die definierte Durchflussrate erreicht und/oder überschreitet, sodass der Atemluftstrom als Kühlluftstrom einsetzbar ist und eine auseichende Kühlung während des beschleunigten Aufladens

11. Beatmungsvorrichtung (100) nach einem der vorhergehenden Ansprüche, wobei wenigstens eine Steuereinrichtung (6) dazu geeignet und ausgebildet ist, eine Durchflussrate des Kühlluftstroms in Abhängigkeit wenigstens einer vorgesehenen Ladeleistung der Ladeeinrichtung (3) einzustellen und/oder wenigstens eine Ladeleistung in Abhängigkeit wenigstens einer vorgesehenen Durchflussrate des Kühlluftstroms einzustellen.

12. Beatmungsvorrichtung (100) nach dem vorhergehenden Anspruch, wobei die Steuereinrichtung (6) dazu geeignet und ausgebildet ist, wenigstens eine Temperatur der Energiespeichereinrichtung (2) zu erfassen und in Abhängigkeit der erfassten Temperatur die Ladeeinrichtung (3) und/oder die Kühleinrichtung (4), insbesondere zumindest eine Gebläseeinrichtung (14, 24), wenigstens teilweise zu regeln.

13. Beatmungsvorrichtung (100) nach einem der vorhergehenden Ansprüche, wobei die Steuereinrichtung (6) dazu geeignet und ausgebildet ist, Leistungs-Bedarfe anderer Leistungsverbraucher, wie beispielsweise Bedieneinrichtung, Display oder Anfeuchter, sowie die Temperatur der Energiespeichereinrichtung (2) zu ermitteln und/oder zu berücksichtigen, und unter Berücksichtigung der zulässigen Temperatur der Energiespeichereinrichtung (2) zumindest zeitweise eine für die Ladung der Energiespeichereinrichtung (2) maximal verfügbare Leistung bereit zu stellen.

## Claims

1. A respiratory device (100), comprising at least one respirator (1) having at least one respiratory apparatus (11), having a blower apparatus (14) for generating at least one respiratory air flow for respiratory therapy and having at least one rechargeable energy storage apparatus (2) for providing the energy required for operation and having at least one charging apparatus (3) for charging the energy storage apparatus (2), and having a control apparatus (6) for regulating the blower apparatus (14), wherein the respiratory device comprises at least one cooling apparatus (4) having the blower apparatus (14) for generating at least one cooling air flow in order to cool the energy storage apparatus (2) during a charging process such that heat can be dissipated from the energy storage apparatus (2) by means of the cooling air flow, wherein the cooling air flow is provided by the blower apparatus (14) which also generates the respiratory air flow for the respiration, **characterized in that** the control apparatus (6) is configured to regulate the blower apparatus (14) such that the air volume flow continues during the charging process and escapes at the respiratory air outlet (61) of the respirator (1) or at the patient's end of the breathing tube (101).

2. The respiratory device (100) according to the preceding claim, wherein the energy storage apparatus (2) comprises at least one cell unit (12) and at least one wall (22) coupled in a heat-conducting manner to the cell unit (12), and wherein heat can be dissipated from the wall (22) by means of the cooling air flow.

3. The respiratory device (100) according to any one of the preceding claims, comprising at least one transport container (5), in particular a pocket (15), for receiving the respirator (1), wherein the transport container (5) provides at least one inlet (25) and at least one outlet (35) for the cooling air flow and at least one flow connection (45) for the cooling air flow between the inlet (25) and the outlet (35), wherein the flow connection (45) runs at least in sections along at least one part of the energy storage apparatus (2).

4. The respiratory device (100) according to the preceding claim, wherein the energy storage apparatus (2) comprises at least one external battery unit (32) which can be joined to the respirator (1), and wherein the external battery unit (32) can be received in the transport container (5), and wherein the flow connection (45) runs at least in sections along at least one part of the external battery unit (32).

5. The respiratory device (100) according to any one of the preceding claims, wherein the respirator (1) and the external battery unit (32) remain in the transport container during the charging process.

6. The respiratory device (100) according to any one of the two preceding claims, wherein the energy storage apparatus (2) comprises at least one internal battery unit (42) which can be received in the respirator (1), and wherein the flow connection (45) runs at least in sections along at least one wall (22) of the internal battery unit (32), which is directed towards an outside (21) of the respirator (1) and/or runs along at least one outwardly directed appliance portion (31) of the respirator (1), wherein the appliance portion (31) is coupled in a heat-conducting manner to the wall (22) of the internal battery unit (42).

7. The respiratory device (100) according to any one of the preceding claims, wherein the internal battery unit (42) comprises at least one wall (22) directed into an interior (41) of the respirator (1), and wherein heat can be dissipated from said wall (22) by means of a cooling air flow.

8. The respiratory device (100) according to the preceding claim, wherein the internal battery unit (42) comprises at least one wall (22) directed into an interior (41) of the respirator (1), and wherein heat can be dissipated from said wall (22) by means of at least one further cooling air flow, and wherein the cooling apparatus (4) comprises at least one further blower apparatus (24) in order to generate the further cooling air flow.

9. The respiratory device (100) according to any one of the preceding claims, comprising at least one control apparatus (6) which is adapted and configured, in the event of the respiratory apparatus (11) not operating, to conduct at least one charging mode by means of the charging apparatus (3) in order to accelerate the charging of the energy storage apparatus (2) and, to this end, to generate at least one cooling flow having at least one defined flow rate by means of the blower apparatus (14), in order to guarantee sufficient cooling during the accelerated charging.

10. The respiratory device (100) according to the preceding claim, wherein the control apparatus (6) is adapted and configured to then also carry out the charging mode in order to accelerate the charging of the energy storage apparatus (2) during the operation of the respiratory apparatus (11), if a flow rate of the generated respiratory air flow attains and/or exceeds the defined flow rate such that the respiratory air flow can be used as a cooling air flow and guarantees sufficient cooling during the accelerated charging.

11. The respiratory device (100) according to any one of the preceding claims, wherein at least one control apparatus (6) is adapted and configured to adjust a flow rate of the cooling air flow as a function of at least one provided charging power of the charging apparatus (3) and/or to adjust at least one charging power as a function of at least one provided flow rate of the cooling air flow.

12. The respiratory device (100) according to the preceding claim, wherein the control apparatus (6) is adapted and configured to detect at least one temperature of the energy storage apparatus (2) and, as a function of the detected temperature, to at least partially regulate the charging apparatus (3) and/or the cooling apparatus (4), in particular at least one blower apparatus (14, 24).

13. The respiratory device (100) according to any one of the preceding claims, wherein the control apparatus (6) is adapted and configured to establish and/or to consider power requirements of other power consumers such as, for example, operating apparatus, display or moistener, as well as the temperature of the energy storage apparatus (2), and taking account of the permissible temperature of the energy storage apparatus (2), to provide at least temporarily a maximum available power for the charging of the energy storage apparatus (2).

## Revendications

1. Dispositif de ventilation (100) comprenant au moins un respirateur (1) avec au moins un système de ventilation (11), avec un système de soufflante (14) pour générer au moins un flux d'air respiratoire destiné à une thérapie respiratoire et avec au moins un système de stockage d'énergie rechargeable (2) pour fournir l'énergie nécessaire au fonctionnement et avec au moins un système de charge (3) pour recharger le système de stockage d'énergie (2), et avec un système de commande (6) pour réguler le système de soufflante (14), dans lequel au moins un système de refroidissement (4) avec le système de soufflante (14) pour générer au moins un flux d'air de refroidissement est compris afin de refroidir le système de stockage d'énergie (2) durant un processus de charge, de sorte que de la chaleur peut être dissipée du système de stockage d'énergie (2) au moyen du flux d'air de refroidissement, dans lequel le flux d'air de refroidissement est fourni par le système de soufflante (14), lequel génère aussi le flux d'air respiratoire destinée à la ventilation artificielle, **caractérisé en ce que** le système de commande (6) est conçu pour réguler le système de soufflante (14) de manière à ce que le débit volumique d'air dure pendant le processus de charge et qu'il s'écoule à la sortie d'air respiratoire (61) du respirateur (1) ou à l'extrémité du tuyau de respiration (101) du côté du patient.

2. Dispositif de ventilation (100) selon la revendication précédente, dans lequel le système de stockage d'énergie (2) comprend au moins une unité à cellules (12) et au moins une paroi (22) couplée à l'unité à cellules (12) de manière thermoconductrice et dans lequel de la chaleur peut être dissipée de la paroi (22) au moyen du flux d'air de refroidissement.

3. Dispositif de ventilation (100) selon l'une des revendications précédentes, comprenant au moins un récipient de transport (5), en particulier une sacoche (15), pour recevoir le respirateur (1), dans lequel le récipient de transport (5) prévoit au moins une entrée (25) et au moins une sortie (35) du flux d'air de refroidissement et au moins une connexion fluidique (45) du flux d'air de refroidissement entre .l'entrée (25) et la sortie (35), dans lequel la connexion fluidique (45) s'étend au moins par endroits le long au moins d'une partie du système de stockage d'énergie (2) .

4. Dispositif de ventilation (100) selon la revendication précédente, dans lequel le système de stockage d'énergie (2) comprend au moins un bloc d'accumulateurs externe (32) pouvant être relié au respirateur (1) et dans lequel le bloc d'accumulateurs externe (32) peut être logé dans le récipient de transport (5) et dans lequel la connexion fluidique (45) s'étend au moins par endroits le long au moins d'une partie du bloc d'accumulateurs externe (32).

5. Dispositif de ventilation (100) selon l'une des revendications précédentes, dans lequel le respirateur (1) et le bloc d'accumulateurs externe (32) restent, dans le récipient de transport durant le processus de charge.

6. Dispositif de ventilation (100) selon l'une des deux revendications précédentes, dans lequel le système de stockage d'énergie (2) comprend au moins un bloc d'accumulateurs interne (42) pouvant être logé dans le respirateur (1) et dans lequel la connexion fluidique (45) s'étend au moins par endroits le long au moins d'une paroi (22) du bloc d'accumulateurs interne (42), orientée vers une face extérieure (21) du respirateur (1) et/ou le long au moins d'une partie d'appareil (31) orientée vers l'extérieur du respirateur (1), dans lequel la partie d'appareil (31) est couplée à la paroi (22) du bloc d'accumulateurs interne (42) de manière thermoconductrice.

7. Dispositif de ventilation (100) selon l'une des revendications précédentes, dans lequel le bloc d'accumulateurs interne (42) comprend au moins une paroi (22) orientée dans un espace intérieur (41) du respirateur (1) et dans lequel de la chaleur peut être dissipée de cette paroi (22) au moyen d'un flux d'air de refroidissement.

8. Dispositif de ventilation (100) selon la revendication précédente, dans lequel le bloc d'accumulateurs interne (42) comprend au moins une paroi (22) orientée dans un espace intérieur (41) du respirateur (1) et dans lequel de la chaleur peut être dissipée de cette paroi (22) au moyen au moins d'un autre flux d'air de refroidissement et dans lequel le système de refroidissement (4) comprend au moins un autre système de soufflante (24) pour générer l'autre flux d'air de refroidissement.

9. Dispositif de ventilation (100) selon l'une des revendications précédentes, comprenant au moins un système de commande (6), lequel se prête à l'emploi et est conçu pour effectuer au moins un mode de charge accélérant la charge du système de stockage d'énergie (2) au moyen du système de charge (3) en cas de non-fonctionnement du système de ventilation (11) et en plus pour générer au moins un flux de refroidissement avec au moins un débit défini au moyen du système de soufflante (14) afin de garantir un refroidissement suffisant durant la charge accélérée.

10. Dispositif de ventilation (100) selon la revendication précédente, dans lequel le système de commande (6) se prête à l'emploi et est conçu pour exécuter le mode de charge accélérant la charge du système de stockage d'énergie (2) même en cas de fonctionnement du système de ventilation (11) (11) chaque fois qu'un débit du flux d'air respiratoire alors généré atteint et/ou dépasse le débit défini, de sorte que le flux d'air respiratoire est utilisable comme flux d'air de refroidissement et qu'il garantit un refroidissement suffisant durant la charge accélérée.

11. Dispositif de ventilation (100) selon l'une des revendications précédentes, dans lequel au moins un système de commande (6) se prête à l'emploi et est conçu pour ajuster un débit du flux d'air de refroidissement en fonction d'au moins une puissance de charge prévue du système de charge (3) et/ou pour ajuster au moins une puissance de charge en fonction d'au moins un débit prévu du flux d'air de refroidissement.

12. Dispositif de ventilation (100) selon la revendication précédente, dans lequel le système de commande (6) se prête à l'emploi et est conçu pour saisir au moins une température du système de stockage d'énergie (2) et pour régler au moins en partie le système de charge (3) et/ou le système de refroidissement (4), en particulier au moins un système de soufflante (14, 24), en fonction de la température saisie.

13. Dispositif de ventilation (100) selon l'une des revendications précédentes, dans lequel le système de commande (6) se prête à l'emploi et est conçu pour déterminer et/ou prendre en considération les besoins en puissance d'autres consommateurs d'énergie tels que système de manipulation, afficheur ou humidificateur, ainsi que la température du système de stockage d'énergie (2), et pour fournir au moins par moments une puissance maximale disponible en vue de charger le système de stockage d'énergie (2) compte tenu de la température admissible du système de stockage d'énergie (2).
